# EUROPÄISCHE PATENTSCHRIFT

(11) **EP 2 363 412 B1**
(45) Veröffentlichungstag und Bekanntmachung des Hinweises auf die Patenterteilung: **10.06.2015**
(21) Anmeldenummer: 10181077.8
(22) Anmeldetag: 11.10.2006
(51) Int. Cl.: C07K 14/52

(54) **Antagonisten gegen die Interaktion von PF4 und RANTES**
Antagonists against interaction of PF4 and RANTES
Antagonistes Utilises Contre L'Interaction de PF4 et de RANTES

(30) Priorität: 14.10.2005 DE 102005049637
(43) Veröffentlichungstag der Anmeldung: 07.09.2011
(62) Teilanmeldung aus: 06806162.1
(73) Patentinhaber: RWTH Aachen, 52056 Aachen (DE)
(72) Erfinder: Weber, Christian, 52074 Aachen (DE); Von Hundelshausen, Philipp, 82024 Taufkirchen (DE); Koenen, Rory, München (DE)
(74) Vertreter: Williams, Richard Andrew Norman

(56) Entgegenhaltungen:
- WO-A-00/27880
- WO-A2-2010/042548
- DE-A1- 10 014 516
- VON HUNDELSHAUSEN PHILIPP ET AL: "Heterophilic interactions of platelet factor 4 and RANTES promote monocyte arrest on endothelium", BLOOD BLOOD, Bd. 105, Nr. 3 1. Februar 2005 (2005-02-01), Seiten 924-930, XP002417923, ISSN: 0006-4971

### SEQUENCE LISTING

<110> RWTH Aachen
<120> Antagonisten gegen die Interaktion von PF4 und RANTES
<130> UD 40022 / SAM
<160> 15
<170> PatentIn version 3.3
<210> 1
   <211> 22
   <212> PRT
   <213> Artificial
<220>
   <223> Antagonist
<220>
   <221> misc-feature
   <222> (2)..(3)
   <223> Xaa can be any naturally occurring amino acid
<220>
   <221> misc_feature
   <222> (9)..(21)
   <223> Xaa can be any naturally occurring amino acid
<400> 1
   Cys Xaa Xaa Tyr Phe Tyr Thr Ser Xaa Xaa Xaa Xaa Xaa Xaa Xaa Xaa
   1               5                   10                  15
   Xaa Xaa Xaa Xaa Xaa Cys
               20
<210> 2
   <211> 22
   <212> PRT
   <213> Artificial
<220>
   <223> Antagonist
<400> 2
   Cys Lys Glu Tyr Phe Tyr Thr Ser Gly Lys Cys Ser Asn Pro Ala Val
   1               5                   10                  15
   Val Phe Val Thr Arg Cys
               20
<210> 3
   <211> 22
   <212> PRT
   <213> Artificial
<220>
   <223> Antagonist
<400> 3
   Cys Lys Glu Tyr Phe Tyr Thr Ser Gly Lys Ser Ser Asn Pro Gly Ile
   1               5                   10                  15
   Val Phe Ile Thr Arg Cys
               20
<210> 4
   <211> 20
   <212> PRT
   <213> Artificial
<220>
   <223> Antagonist
<400> 4
   Cys Glu Tyr Phe Tyr Thr Ser Gly Lys Ser Ser Asn Pro Gly Ile Val
   1               5                   10                  15
   Phe Ile Thr Cys
               20
<210> 5
   <211> 20
   <212> PRT
   <213> Artificial
<220>
   <223> Antagonist
<400> 5
   Cys Glu Tyr Phe Tyr Thr Ser Gly Lys Ser Ser Asn Tyr Gly Ile Val
   1               5                   10                  15
   Phe Ile Thr Cys
               20
<210> 6
   <211> 20
   <212> PRT
   <213> Artificial
<220>
   <223> Antagonist
<400> 6
   Cys Glu Tyr Phe Tyr Thr Ser Ser Lys Ser Ser Asn Tyr Gly Ile Val
   1               5                   10                  15
   Phe Ile Thr Cys
               20
<210> 7
   <211> 19
   <212> PRT
   <213> Artificial
<220>
   <223> Antagonist
<400> 7
   Cys Tyr Phe Tyr Thr Ser Ser Lys Ser Ser Asn Pro Gly Ile Val Phe
   1               5                   10                  15
   Ile Thr Cys
<210> 8
   <211> 66
   <212> DNA
   <213> Artificial
<220>
   <223> Sequence coding for Antagonist
<400> 8
   tgcaaggaat atttctacac ttccgggaaa tcctccaatc ctggaattgt gttcatcact 60
   agatgt 66
<210> 9
   <211> 66
   <212> DNA
   <213> Artificial
<220>
   <223> Sequence coding for Antagonist
<400> 9
   tgcaaggaat atttctacac ttccgggaaa tgttccaatc ctgccgtggt gttcgtcact 60
   agatgt 66
<210> 10
   <211> 60
   <212> DNA
   <213> Artificial
<220>
   <223> Sequence coding for Antagonist
<400> 10
   tgcgaatatt tctacacttc cgggaaatcc tccaatcctg gaattgtgtt catcacttgt 60
<210> 11
   <211> 60
   <212> DNA
   <213> Artificial
<220>
   <223> Sequence coding for Antagonist
<400> 11
   tgcgaatatt tctacacttc cgggaaatcc tccaattacg gaattgtgtt catcacttgt 60
<210> 12
   <211> 60
   <212> DNA
   <213> Artificial
<220>
   <223> sequence coding for Antagonist
<400> 12
   tgcgaatatt tctacacttc ctctaaatcc tccaattacg gaattgtgtt catcacttgt 60
<210> 13
   <211> 57
   <212> DNA
   <213> Artificial
<220>
   <223> Sequence coding for Antagonist
<400> 13
   tgctatttct acacttcctc taaatcctcc aatcctggaa ttgtgttcat cacttgt 57
<210> 14
   <211> 9
   <212> PRT
   <213> Artificial
<220>
   <223> Control peptide
<400> 14
   Lys Glu Tyr Phe Tyr Thr Ser Gly Lys
   1               5
<210> 15
   <211> 22
   <212> PRT
   <213> Artificial
<220>
   <223> Antagonist
<400> 15
   Cys Lys Glu Tyr Phe Tyr Thr Ser Ser Lys Ser Ser Asn Leu Ala Val
   1               5                   10                  15
   Val Phe Val Thr Arg Cys
               20

## Beschreibung

Die Erfindung betrifft Polypeptide, deren pharmakologisch akzeptable Salze, Derivate und/oder Konjugate, deren Verwendung zur Herstellung eines Arzneimittels sowie Arzneimittel. Die Polypeptide sind geeignet zur Behandlung von Erkrankungen, die im Zusammenhang mit einer Rekrutierung von Monozyten stehen.

Die Erfindung betrifft Polypeptide, deren pharmakologisch akzeptable Salze, Derivate und/oder Konjugate, deren Verwendung zur Herstellung eines Arzneimittels sowie Arzneimittel. Die Polypeptide sind geeignet zur Behandlung von Erkrankungen, die im Zusammenhang mit einer Rekrutierung von Monozyten stehen.

Die Arteriosklerose arterieller Gefäße bildet den morphologischen Hintergrund kardiovaskulärer Erkrankungen. Hierbei ist die initiale Rekrutierung von Monozyten von entscheidender Bedeutung für die Genese der arteriosklerotischen Frühläsion. Die Anheftung der Monozyten auf dem Endothel, der sogenannte Monozytenarrest, steht am Beginn der Pathogenese kardiovaskulärer Erkrankungen wie Arteriosklerose, Stenosen und Thrombosen. Es ist bekannt, dass Chemokine wie RANTES (regulated on activation, normal T cell expressed and secreted) als Signalmoleküle mit diesen Vorgängen in Verbindung stehen.

Im Stand der Technik bekannte Primär- und Sekundärprävention sind vor allem eine Lipidsenkende Behandlung sowie die Hemmung der Thrombozytenaggregation und - aktivierung durch Medikamente wie Aspirin oder Clopidogrel. Der Nachteil der Behandlung mit diesen Medikamenten ist zum einen, dass diese eine nur geringe Spezifität zeigen, zum anderen, dass diese Medikamente gravierende Nebenwirkungen wie Myopathien und eine erhöhte Blutungsgefahr mit sich bringen.

Weiterhin ist im Stand der Technik bekannt, RANTES-Peptidantagonisten einzusetzen. Beispielsweise offenbart die DE 100 14 516 A1 die Verwendung von metRANTES als Antagonist gegen den RANTES-Rezeptor CCR1. Nachteilig bei der Verwendung dieses Antagonisten ist, dass Chemokine als Signalmoleküle an einer Vielzahl physiologischer Prozesse beteiligt sind, so dass durch die Verwendung eines solchen Antagonisten eine unabsehbare Zahl an physiologischen Prozessen beeinflusst wird und zahlreiche Nebenwirkungen und Folgeerscheinungen auftreten können.

Die Aufgabe der vorliegenden Erfindung bestand darin, Mittel zur Verfügung zu stellen, die wenigstens einen der Nachteile des Standes der Technik überwinden. Insbesondere bestand die Aufgabe der vorliegenden Erfindung darin, Mittel zur Verfügung zu stellen, die eine verbesserte Spezifität aufweisen.

Dementsprechend stellt die vonlugende erfindung ein Polypeptide, bestehend aus der sequenz CKEYFYTSSKSSNLAVVFVTRC nach Anspruch 1 angegeben.

Vorteilhafter Weise sind die erfindungsgemäßen Polypeptide als Antagonist gegen die Interaktion zwischen RANTES und Platelet Factor 4 geeignet.

Unter dem Begriff "Antagonist gegen die Interaktion zwischen RANTES und Platelet Factor 4" sind im Sinne der vorliegenden Erfindung Peptide, Proteine oder andere Verbindungen zu verstehen, die als Antagonist gegen die Interaktion zwischen den Chemokinen RANTES und Platelet Factor 4 fungieren können.

Es wurde überraschend gefunden, dass die erfindungsgemäßen Polypeptide eine spezifische Wirkung auf die durch die Interaktion der Chemokine RANTES und Platelet Factor 4 (PF4) vermittelte Rekrutierung von Monozyten aufweisen können. Von besonderem Vorteil ist hierbei, dass die erfindungsgemäßen Polypeptide keine oder lediglich geringe Auswirkungen auf die zahlreichen Funktionen der Chemokine zeigen. Insbesondere ist von Vorteil, dass eine selektive Blockierung der Rekrutierung von Monozyten beispielsweise auf Endothel bewirkt werden kann.

Der Begriff der "Rekrutierung von Monozyten" umfasst im Sinne der vorliegenden Erfindung die Bedeutung einer Ein- bzw. Auswanderung von Monozyten durch das Endothel, deren Anheftung sowie Ausbreitung beispielsweise in endotheliale Spalten. Die Anheftung der Monozyten wird ebenfalls als Monozytenadhäsion bezeichnet, bzw. als Monozytenarrest wenn die Anheftung wie unter physiologischen Bedingungen in Scherfluss erfolgt, beispielsweise in Blutkapillaren, mikrovaskulären oder arteriellen Strombahnen.

Es ist von grossem Vorteil, dass die erfindungsgemäßen Polypeptide eine hohe Spezifität zur Verfügung stellen können, und keine oder lediglich geringe Nebenwirkungen auf die zahlreichen, durch die Chemokine RANTES und PF4 vermittelten Stoffwechselvorgänge, beispielsweise des Immun- oder Gerinnungssystems, zeigen. Insbesondere kann durch die Verabreichung der erfindungsgemäßen Polypeptide ein Blutungsrisiko wie bei herkömmlicher Medikation bei kardiovaskulären Erkrankungen vermieden werden.

"C" steht vorliegend entsprechend dem üblichen hier verwendeten Ein-Buchstaben-Code der Aminosäuren für die Aminosäure Cystein, entsprechend stehen "Y" für Tyrosin, "F" für Phenylalanin, "T" für Threonin und "S" für Serin.

Das erfindungsgemäße Polypeptid weist am aminoterminalen und am carboxyterminalen Ende jeweils einen Cysteinrest auf, die eine Cyclisierung des Polypeptids zur Verfügung stellen können. Von besonderem Vorteil ist, dass ein cyclisiertes Polypeptid eine verbesserte Stabilität aufweist. Das erfindungsgemäße Polypeptid kann eine längere Wirksamkeit aufweisen und ist entsprechend in geringerer Menge verwendbar.

Unter dem Begriff "Polypeptid" sind im Sinne der vorliegenden Erfindung synthetische oder nicht-synthetische Peptidverbindungen zu verstehen, wie auch gereinigte, modifizierte Fragmente natürlicher Proteine, native Formen oder rekombinante Peptide oder Proteine. Ebenfalls umfasst der Begriff "Polypeptid" im Sinne der vorliegenden Erfindung pharmakologisch akzeptable Salze, pharmakologisch akzeptable Derivate und/oder Konjugate des entsprechenden Polypeptids.

Bevorzugte pharmakologisch akzeptable Derivate sind beispielsweise Ester, Amide, N-acyl- und/der O-acyl-Derivate, carboxylierte, acetylierte, phosphorylierte und/oder glykosylierte Polypeptide. Bevorzugte Konjugate sind beispielsweise Zucker- oder Polyethylenglycol-Konjugate, biotinylierte, radioaktiv oder fluoreszenzmarkierte Polypeptide.

Insbesondere ist überraschend, dass eine Aminosäuresequenz dieser Länge eine Hemmung des Monozytenarrestes bewirken kann.

Ganz besonders bevorzugt weist das Polypeptid 22 Aminosäuren auf. Der Begriff "Anzahl an Aminosäuren" umfasst im Sinne der Erfindung selbstverständlich auch die Bedeutung der Länge der Aminosäurensequenz des Polypeptids.

Ein Vorteil des Polypeptids liegt in bevorzugten Ausführungsformen darin, dass diese Polypeptide bevorzugt eine verbesserte Stabilität aufweisen. Dies ermöglicht, dass die Polypeptide in höherem Ausmaß ihren Wirkort erreichen können und stabile Interaktionen mit Proteinen oder Peptidverbindungen eingehen können.Insbesondere ermöglicht eine erhöhte Stabilität des Polypeptids, dass dieses in vivo und in vitro verwendbar ist. Ein weiterer Vorteil des Polypeptids liegt in bevorzugten Ausführungsformen darin, dass das Polypeptid eine verbesserte Lösbarkeit in Wasser aufweist. Eine erhöhte Lösbarkeit kann insbesondere dazu führen, dass das Polypeptid einfacher und leichter applizierbar ist.

Weiterhin kann vorgesehen sein, dass die jeweiligen L-Aminosäuren durch D-Aminosäuren ersetzt sind. Hierdurch kann eine weitere Erhöhung der Stabilität erreicht werden.

Die Polypeptide sind nach üblichen Methoden der Peptidsynthese herstellbar.

Vorteilhafter Weise sind die erfindungsgemäßen Polypeptide als Antagonist gegen die Interaktion zwischen RANTES und Platelet Factor 4 geeignet. Insbesondere können die erfindungsgemäßen Polypeptide eine Inhibition der Wechselwirkung zwischen RANTES und Platelet Factor 4 bewirken.

Die erfindungsgemäßen Polypeptide, deren pharmakologisch akzeptablen Salze, Derivate und/oder Konjugate, sind als Antagonisten gegen die Interaktion zwischen RANTES und Platelet Factor 4 verwendbar.

Aufgrund ihrer vorteilhaften Eigenschaften sind die erfindungsgemäßen Polypeptide zur Verwendung als Arzneimittel geeignet.

Ein weiterer Gegenstand der Erfindung betrifft die Verwendung der erfindungsgemäßen Polypeptide, insbesondere der bevorzugten Ausführungsformen, zur Herstellung eines Arzneimittels.

Die erfindungsgemäßen Polypeptide sind entsprechend üblichen Methoden verabreichbar, bevorzugt ist eine parentale Verabreichung beispielsweise eine orale Verabreichung, dermale Verabreichung, subkutane Verabreichung und/oder intravenöse Verabreichung. Beispielsweise können die Polypeptide mittels ex vivo-Applikationen verabreicht werden, beispielsweise vor Implantationen eines Gefäßinterponats, oder mittels intraversaler Applikation, beispielsweise vor oder nach einer Katheterintervention oder Stentimplantation.

Für eine solche Anwendung ist eine vorzugsweise gute Lösbarkeit der Polypeptide in Wasser von großem Vorteil.

Es kann neben einer zeitlich befristeten oder akuten Therapie weiterhin bevorzugt sein, dass die erfindungsgemäßen Polypeptide über einen längeren Zeitraum verabreicht werden. Die erfindungsgemäßen Polypeptide können ebenfalls in einer Form der verlängerten oder verzögerten Freisetzung verabreichbar sein, beispielsweise in Form von Depotinjektionen oder osmotischen Pumpen.

Das erfindungsgemäße Polypeptid kann ebenfalls in Form einer Nukleinsäure kodierend für das jeweilige Polypeptid verabreicht werden. Hierbei kann das Nukleinsäuremolekül in üblichen Vektoren enthalten sein. Vorzugsweise wird eine DNA-Sequenz kodierend für das jeweilige Polypeptid verabreicht. Ebenfalls kann vorgesehen sein, die für ein erfindungsgemäßes Polypeptid kodierende RNA zu verabreichen.

Weiterhin ist bekannt, dass Veränderungen in der Sequenz der Nukleinsäuren vorhanden sein können, zum Beispiel durch die Degenerierung des genetischen Codes.

Bevorzugte Dosierungen der erfindungsgemässen Polypeptide liegen für die Verabreichung an Menschen im Bereich von > 10 mg/Tag/75 kg Körpergewicht bis ≤ 1000 mg/Tag/75 kg Körpergewicht, bevorzugt im Bereich von ≥ 50 mg/Tag/75 kg Körpergewicht bis ≤ 200mg/Tag/75 kg Körpergewicht, vorzugsweise im Bereich von 150 mg/Tag/75 kg Körpergewicht.

Die erfindungsgemässen Polypeptide, deren pharmakologisch akzeptable Salze, Derivate und/oder Konjugate und/oder Nukleinsäuren sind insbesondere zur therapeutischen und/oder prophylaktischen Behandlung, Diagnose und/oder Therapie von Erkrankungen, die mit einer Rekrutierung von Monozyten in Zusammenhang stehen, verwendbar. Zu diesen Erkrankungen zählen beispielsweise kardiovaskuläre und/oder entzündliche Erkrankungen, insbesondere Arteriosklerose, Stenosen, Bluthochdruck und/oder Transplantabstoßungsreaktionen.

Insbesondere sind die erfindungsgemäßen Polypeptide und/oder Nukleinsäuren zur Behandlung von Säugetieren insbesondere des Menschen verwendbar.

Die erfindungsgemäßen Polypeptide können die Adhäsion von Monozyten am Endothel positiv beeinflussen. Insbesondere wurde überraschend gefunden, dass insbesondere bevorzugte Ausführungsformen der erfindungsgemäßen Polypeptide die Verstärkung eines solchen Monozytenarrests vermittelt durch die heterophile Interaktion von RANTES und PF4 hemmen können. Vorteilhafterweise können insbesondere bevorzugte Ausführungsformen der erfindungsgemäßen Polypeptide in Experimenten eine verbesserte Hemmung der Verstärkung des Monozytenarrests vermittelt durch die heterophile Interaktion von RANTES und PF4 zeigen, als bislang bekannte Protein- oder Peptidverbindungen.

Ein besonderer Vorteil der erfindungsgemäßen Polypeptide kann dadurch verwirklicht werden, dass durch die Verabreichung der Polypeptide die Ausbildung einer Arteriosklerose, postoperativen oder postinterventionellen Restenosen, beispielsweise nach Ballondilatation, Atherektomie oder Bypass-Operationen vermindert oder verhindert werden kann. Von besonderem Vorteil ist, dass die erfindungsgemäßen Polypeptide auch bei fortgeschrittener bzw. klinischer Erkrankung bzw. morphologischen arteriosklerotischen Veränderungen eine weitere Rekrutierung von Monozyten auf dem aktivierten Endothel verhindern oder vermindern können.

Von besonderem Vorteil ist, dass die erfindungsgemäßen Polypeptide insbesondere zur prophylaktischen Behandlung beispielsweise bei Bluthochdruckrisikopatienten verwendbar sind. Eine solche prophylaktische Verwendung wird vorteilhafterweise insbesondere dadurch ermöglicht, dass die erfindungsgemäßen Polypeptide keine oder lediglich geringe Auswirkungen auf allgemeine Chemokin vermittelte Prozesse zeigen.

Ein weiterer Gegenstand der Erfindung betrifft entsprechend die Verwendung der erfindungsgemäßen Polypeptide, deren pharmakologisch akzeptable Salze, Derivate und/oder Konjugate, zur Herstellung eines Arzneimittels zur therapeutischen und/oder prophylaktischen Behandlung von Erkrankungen ausgewählt aus der Gruppe umfassend:
- Erkrankungen, die mit einer Rekrutierung von Monozyten in Zusammenhang stehen, wie kardiovaskuläre und/oder entzündliche Erkrankungen, insbesondere Arteriosklerose, Atherosklerose, instabile Plaques, Stenosen, Restenosen, Bluthochdruck, Arthritis, Myokarditis, Autoimmunerkrankungen einschließlich Enzephalomyelitiden, entzündliche Darmerkrankungen, Reperfusionschäden nach Infarkten, beispielsweise myokardialen oder cerebrovaskulären Infarkten, Transplantabstoßung und/oder Hauterkrankungen wie Psoriasis, und/oder
- Erkrankungen, die mit einer RANTES-abhängigen Rekrutierung anderer Leukozyten-Populationen in Zusammenhang stehen, wie Eosinophile, insbesondere bei allergischen Erkrankungen wie Asthma oder Pneumonitiden.

Insbesondere bei der Behandlung von atherosklerotischen Veränderungen des Menschen kann durch eine Verwendung der erfindungsgemäßen Polypeptide ein vorteilhafter Effekt auf den Krankheitsverlauf erzielt werden. Insbesondere kann eine Verstärkung der arteriosklerotischen Veränderungen durch Monozytenarrest vermindert werden. Ein weiterer Vorteil der erfindungsgemäßen Polypeptide kann sich daraus ergeben, dass diese Abstoßungsreaktionen nach der Transplantation von Organen und/oder Gewebe vermindern oder sogar verhindern können.

Von besonderem Vorteil ist hierbei, dass die erfindungsgemäßen Polypeptide vorzugsweise keine oder nur geringe Nebenwirkungen verursachen.Dies ermöglicht, dass die erfindungsgemäßen Polypeptide prophylaktisch verabreichbar sind. Weiterhin ist von besonderem Vorteil, dass durch die Spezifität der erfindungsgemäßen Polypeptide eine Beeinflussung weiterer Stoffwechselvorgänge vermeidbar ist, so dass eine prophylaktische Verabreichung beispielsweise bei Bluthochdruck-Risiko-Patienten oder bei der Prophylaxe arteriosklerotischer Veränderungen ermöglicht wird.

Arzneimittel umfassend erfindungsgemäße Polypeptide sind insbesondere zur Behandlung in vivo beispielsweise des Menschen verwendbar. Eine bevorzugte Verwendung der Arzneimittel umfassend erfindungsgemäße Polypeptide sind die therapeutische und/oder prophylaktische Behandlung von Erkrankungen, die mit einer Rekrutierung von Monozyten in Zusammenhang stehen, wie kardiovaskuläre und/oder entzündliche. Erkrankungen, insbesondere Arteriosklerose, Atherosklerose, instabile Plaques, Stenosen, Restenosen, Bluthochdruck, Arthritis, Myokarditis, Autoimmunerkrankungen einschließlich Enzephalomyelitiden, entzündliche Darmerkrankungen, Reperfusionschäden nach Infarkten, beispielsweise myokardialen oder cerebrovaskulären Infarkten, Transplantabstoßung und/oder Hauterkrankungen wie Psoriasis, und/oder Erkrankungen, die mit einer RANTES-abhängigen Rekrutierung anderer Leukozyten-Populationen in Zusammenhang stehen, wie Eosinophile, insbesondere bei allergischen Erkrankungen wie Asthma oder Pneumonitiden.

Ebenfalls Gegenstand der Erfindung sind Arzneimittel umfassend Nukleinsäuren kodierend für erfindungsgemäße Polypeptide. Hierbei kann das Nukleinsäuresmolekül in üblichen Vektoren enthalten sein.

Der Begriff "Mittel gegen Monozytenarrest" hat im Sinne dieser Erfindung die Bedeutung, dass das Mittel Erkrankungen, die mit einem Monozytenarrest, der Anheftung von Monozyten beispielsweise auf Endothel, in Zusammenhang stehen, positiv beeinflußen kann. Insbesondere kann die Ausbildung von arteriosklerotischen Plaques vermindert oder sogar verhindert werden. Vorzugsweise kann die Verwendung der erfindungsgemäßen Polypeptide dazu fu"hren, dass die Rekrutierung von Monozyten und/oder deren Anheftung auf aktiviertem Endotel insbesondere auch auf arteriosklerotischen Plaques bzw.Neointima vermindert werden kann und/oder vollständig oder nahezu vollständig vermieden werden kann. Beispiele, die der Veranschaulichung der vorliegenden Erfindung dienen, sind nachstehend angegeben.

### Material und Methoden

### Zellkultur

Endothelzellen aus der humanen Nabelschnur (HUVEC, human umbilical vein endothelial cells, PromoCell, Heidelberg) wurden in Endothelial Cell Growth Medium (PromoCell, Heidelberg) kultiviert und nach 2 bis 4 Passagen benutzt.

Monozytäre Mono Mac 6-Zellen (MM6, DSMZ) wurden in RPMI 1640 Medium (PAA Laboratories, Pasching, Österreich) unter Zusatz von 10 % fetalem Kälberserum, 2 mM L-Glutamin (Biowhittaker), 1 mM Natriumpyruvat, 50 µg/ml Gentamycin und 9 µg/ml Insulin (MM6-Medium) kultiviert. Die Zellen wurden mit einer Dichte von 2 x 10⁵/ml in 2 ml MM6-Medium in 24 well-Platten ausgesäht und bei 37°C in einer befeuchteten Atmosphäre mit 5% CO2 für 3 bis 4 Tage kultiviert, bevor sie für Experimente benutzt wurden.

### Polypeptide

Polypeptide der Sequenz SEQ ID NO: 3 gemäß der Formel (3), dessen Maus-Ortholog gemäß der Sequenz SEQ ID NO: 15 gemäß der Formel (15) sowie eines Kontrollpeptids der Sequenz SEQ ID NO: 14 gemäß der Formel (14) wurden chemisch mittels t-Boc-basierter Festphasenpeptidsynthese unter Verwendung von 4-Methylbenzhydrylamin-Harz synthetisiert, mittels Umkehrphasen-HPLC gereinigt und gegebenenfalls in 6 M Guanidin-HCl/Tris pH 8 zyklisiert. Die Molekularmasse wurde mittels Elektrospray-Massenspektrometrie bestimmt ( Dawson PE, Kent SB. (2000) Annu Rev Biochem. 69: 923-960, Hackeng TM, Griffin JH, Dawson PE. (1999) Proc Natl Acad Sci USA., Vol 96, S. 10068-10073).

### Beispiel 1 (nicht erfindungsgemäß)

Plasmonresonanzstudien zur Analyse des inhibitorischen Effekts des Polypeptids der Sequenz SEQ ID NO: 3 gemäß der Formel (3) auf die Bildung von Heteroaggregaten von RANTES und PF4

Die Plasmonresonanzstudien wurden unter Verwendung von HBS-EP Puffer (10 mM HEPES, 150 mM NaCl, 0,005 % Tween 20, pH 7,4) durchgeführt.

Zwei Flusszellen eines C1 Chips (Biacore AB, Uppsala, Schweden) wurden durch Injektion von 50 µl Ethyl(dimethylaminopropyl)carbodümid/N-hydroxy-succinimid (0,2 M/0,05 M, Firma Pierce) aktiviert und nachfolgend wurden 20 µl Streptavidin (0,2 mg/ml, Sigma-Aldrich) über die aktivierte Oberfläche perfundiert. Nachfolgend wurde die Oberfläche durch vier aufeinanderfolgende Injektionen von 20 µl Ethylendiamin (1M, pH8, Sigma-Aldrich) inaktiviert.

Am N-Terminus biotinyliertes humanes PF4 (bPF4) wurde chemisch mittels t-Boc-basierter Festphasenpeptidsynthese und nativer chemischer Ligation von PF4 synthetisiert (Dawson PE, Kent SB. (2000) Annu Rev Biochem. 69: 923-960, Hackeng TM, Griffin JH, Dawson PE. (1999) Proc Natl Acad Sci USA., Vol 96, S. 10068-10073). Das bPF4 wurde auf der Dextranoberfläche eines C1 Sensorchips immobilisiert, indem 200 µg/ml bPF4 in HBS-EP über eine der Flusskammern injiziert und 240 Resonanzeinheiten (RU) aufgenommen wurden. Die zweite Flusskammer wurde nicht mit bPF4 behandelt und diente als Referenz.

Die Bindung von RANTES (0,5 µM, rekombinantes humanes RANTES, Peprotech, Rocky Hill, NJ, USA) oder RANTES (0,5 µM), das mit verschiedenen Konzentrationen, 0 µM, 10 µM, 50 µM und 100 µM, des Polypeptids der Sequenz SEQ ID NO: 3 gemäß der Formel (3) in HBS-EP Puffer über Nacht bei Raumtemperatur präinkubiert wurde, an bPF4 wurde mittels Injektion von 15 µl der jeweiligen Peptid/RANTES Mischung und Beobachtung der Bindung für 180 Sekunden ermittelt. Der Kopplungsablauf und die Messungen wurden in einer Vorrichtung Biacore 2000 (Biacore AB) bei einer Flussrate von 5 µl/min durchgeführt. Sensorgramme der RANTES-Bindung wurden mittels der Software BIAevaluation 3.0 (Biacore AB) um unspezifische Hintergrundsignale korrigiert und für jede Injektion wurden Gleichgewichts-Resonanzeinheiten (RU) ermittelt.

Es zeigte sich, dass das Polypeptid der Sequenz SEQ ID NO: 3 gemäß der Formel (3) eine konzentrationsabhängige Inhibition der Interaktion von RANTES und PF4 bewirken konnte, wobei die Bindung von RANTES an immobilisiertes PF4 bis zu 35% in Anwesenheit des Peptids der Sequenz SEQ ID NO: 3 gemäß der Formel (3) bei einer Konzentration von 100 µM reduziert wurde.

### Beispiel 2 nicht erfindungsgemäß

Plasmonresonanzstudien zur Analyse des inhibitorischen Effekts der Polypeptide der Sequenz SEQ ID NO: 3 gemäß der Formel (3), SEQ ID NO: 2 gemäß der Formel (2) und eines Kontrollpeptid auf die Bildung von Heteroaggregaten von RANTES und PF4

In einem weiteren Versuch wurde unter den Bedingungen wie unter Beispiel 1 beschrieben die Bindung von RANTES (0,5 µM) oder RANTES (0,5 µM), das mit 0 µM, 10 µM, 50 µM und 100 µM des Polypeptids der Sequenz SEQ ID NO: 3 gemäß der Formel (3), SEQ ID NO: 2 gemäß der Formel (2) oder eines Kontrollpeptids der Sequenz SEQ ID NO: 14 gemäß der Formel (14) wie nachstehend angegeben:
KEYFYTSGK (14) (SEQ ID NO: 14)
präinkubiert wurde, untersucht.

Es zeigte sich in diesen Versuchen, dass bei einer Konzentration von 10 µM, 50 µM und 100 µM das Polypeptid der Sequenz SEQ ID NO: 3 gemäß der Formel (3) die Interaktion von RANTES und PF4 deutlich effektiver inhibieren konnte, als das Polypeptid der Sequenz SEQ ID NO: 2 gemäß der Formel (2). Das Kontrollpeptid der Sequenz SEQ ID NO: 14 gemäß der Formel (14) zeigte keine nachweisbare Inhibierung bei einer Konzentration von 100 µM.

### Beispiel 3 (nicht erfindungsgemäß)

### Hemmung des Monozytenarrests auf aktiviertem Endothel

Untersucht wurde die Interaktion von monozytären Mono Mac 6-Zellen auf aktivierten Endothelzellen.

Petrischalen mit konfluenten HUVEC-Zellschichten, die mit IL-1β (Interleukin 1β, Peprotech, 10 ng/ml, 12 Stunden) aktiviert wurden, wurden in einer Flusskammer eingebaut. Mono Mac 6 -Zellen (0,5 x 10⁶ Zellen/ml) wurden in ausgewogener Hank-Lösung (HBSS mit 10 mM Hepes (Gibco BRL), pH 7,3, 0,5 % Rinderserumalbumin (Serva)) resuspendiert und auf Eis gehalten. Fünf Minuten vor dem Experiment wurden zu den monozytären MM6-Zellen Ca²⁺ and Mg²⁺ zu einer finalen Konzentration von jeweils 1 mM und 60 nM der Chemokine RANTES (Peprotech, Rocky Hill, NJ, USA) und PF4 (ChromaTec, Greifswald) und jeweils 6 µM der Polypeptide der Sequenz SEQ ID NO: 2 gemäß der Formel (2), der Sequenz SEQ ID NO: 3 gemäß der Formel (3) oder ein Kontrollpeptid der Sequenz SEQ ID NO: 14 gemäß der Formel (14) zugegeben und verwärmt bis 37°C. Die derart vorbehandelten Zellen wurden anschließend bei 1,5 dyn/cm² auf einem Mikroskop des Typs IX 50 der Firma Olympus über die Endothelzellen perfundiert. Die Zahl der Monozyten, die durch Interaktion mit den Endothelzellen adhärent waren, wurde nach 4 Minuten in verschiedenen Feldern mittels Bildanalyse von Aufnahmen einer Videokamera (3CCD, JVC) und Rekorder bestimmt. Die Daten wurden als Mittelwert (n = 5) +- Standardabweichung (p < 0,02) gegen eine Kontrolle ausgewertet.

Es konnte festgestellt werden, dass die Verstärkung des Monozytenarrests durch die heterophile Interaktion von RANTES und PF4 durch das Polypeptid der Sequenz SEQ ID NO: 3 gemäß der Formel (3) signifikant, beispielsweise bis zu 80%, gehemmt werden konnte, während die Hemmung durch das Polypeptid der Sequenz SEQ ID NO: 2 gemäß der Formel (2) schwächer war. Das Kontrollpeptid der Sequenz SEQ ID NO: 14 gemäß der Formel (14) zeigte demgegenüber keine wesentliche Hemmung.

### Beispiel 4

### In vivo-Untersuchungen in einem Mausmodell der Artherosklerose

Als Model der Artherosklerose dienten 9 bis 12 Wochen alte weibliche ApoE-/- littermate-Mäuse (The Jackson Lab, Bar Harbor, Maine, USA). Diese wurden für 12 Wochen fettreich (21 % Fett; Altromin® C1061) ernährt. Während dieser Zeit erhielten zwei Gruppen der Mäuse dreimal wöchentlich intraperitoneale Injektionen von 50 µg des Polypeptids der Sequenz SEQ ID NO: 15 gemäß der Formel (15) wie nachstehend angegeben:
CKEYFYTSSKSSNLAVVFVTRC (15) (SEQ ID NO: 15)
(n = 12 Mäuse) oder eines Kontrollpeptids der Sequenz SEQ ID NO: 14 gemäß der Formel (14) wie nachstehend angegeben:
KEYFYTSGK (14) (SEQ ID NO: 14)
(n = 7 Mäuse) in Salzlösung. Eine nicht behandelte Gruppe der Mäuse (n=12) diente als zusätzliche Kontrolle.

Die Mäuse wurden für histologische Untersuchungen getötet. Während der Versuchsdauer waren die Mäuse gesund und zeigten keine Anzeichen einer Erkrankung. Blutproben wurden zu Beginn und nach Ende der experimentellen Ernährung entnommen.Die Leukozytenzahl wurde haemozytometrisch bestimmt, und die Seren wurden gesammelt und der Cholesterolspiegel mittels des Infinity Cholesterol kits (Thermo Electron, Melbourne, Australien) bestimmt.

Das Ausmaß der Atherosclerose wurde an Aortenwurzeln und thorakoabdominalen Aorten bestimmt, indem Lipidablagerungen mittels Ölrot-O-Färbung angefärbt ( Veillard NR, Kwak B, Pelli G, Mulhaupt F, James RW, Proudfoot AE, Mach F. Antagonism of RANTES receptors reduces atherosclerotic plaque formation in mice.Circ Res. 2004;94:253-61 ) und mittels computerisierter Bildanalyse (Diskus software, Hilgers, Aachen) quantifiziert wurden. Bereiche atherosclerotischer Läsionen wurden in 5 µm-transversalen Schnitten durch Herz und Aortenwurzel bestimmt. Die Bestimmung wurde für jede Aortenwurzel anhand von Lipidgefärbten Bereichen von 6 Schnitten, die 50 µm voneinander entfernt lagen, durchgeführt. Die Bereiche atherosklerotischer Läsionen wurden durch die gesamte Oberfläche der Klappe jedes Schnitts dividiert. Die thorakoabdominale Aorta wurde längs der ventralen Mittellinie geöffnet und die Bereiche der Läsionen wurden in einer en face-Präparation mit mittels Ölrot-O-Färbung angefärbt. Der Anteil der Lipidablagerung wurde berechnet indem der gefärbte Bereich durch die gesamte thorakoabdominale Oberfläche dividiert wurde.

Es konnte festgestellt werden, dass die Mäuse, die mit dem Polypeptid der Sequenz SEQ ID NO: 15 gemäß der Formel (15), dem Maus-Ortholog des Polypeptids der Sequenz SEQ ID NO: 3 gemäß der Formel (3), behandelt wurden, im Vergleich zu den Mäusen, die das Kontrollpeptid erhalten hatten, eine signifikante Verringerung der Entwicklung atherosklerotischer Läsionen zeigten. Weiterhin konnte festgestellt werden, dass der Bereich der Aortawurzel, der Plaques zeigte, relativ zur gesamten Klappenoberfläche ebenfalls in den behandelten Mäusen signifikant vermindert war. Ebenfalls konnte festgestellt werden, dass der Gehalt an Makrophagen in den Läsionen signifikant verringert war.

Somit konnte gezeigt werden, dass durch dass Maus-Ortholog des Polypeptids der Sequenz SEQ ID NO: 3 gemäß der Formel (3) die Entwicklung einer Atherosclerose in vivo verlangsamt werden konnte, und die erfindungsgemäßen Polypeptide somit eine therapeutische Verwendung finden können.

## Patentansprüche

1. Peptid, das aus der Aminosäuresequenz CKEYFYTSSNLAVVFVTRC besteht.

## Claims

1. Peptide, comprising the amino acid sequence CKEYFYTSSNLAVVFVTRC.

## Revendications

1. Peptide, comprenant la séquence d'acide aminé CKEYFYTSSNLAVVFVTRC.
